# EUROPEAN PATENT SPECIFICATION

(11) **EP 2 868 339 B1**
(45) Date of publication and mention of the grant of the patent: **19.10.2016**
(21) Application number: 13191435.0
(22) Date of filing: 04.11.2013
(51) Int. Cl.: A61M 15/00, A61M 11/00, A61M 16/06

(54) **AN AEROSOL DELIVERY SYSTEM**
AEROSOLABGABESYSTEM
SYSTÈME D'ADMINISTRATION D'UN AÉROSOL

(43) Date of publication of application: 06.05.2015
(73) Proprietor: STAMFORD DEVICES LIMITED, Dangan Galway (IE)
(72) Inventor: Eaton-Evans, Jimmy, Galway (IE); Power, Patrick Joseph, County Galway (IE)
(74) Representative: O'Brien, John Augustine

(56) References cited:
- US-A- 3 518 989
- US-A- 5 540 221
- US-A- 5 617 844
- US-A- 6 138 668
- US-A1- 2007 267 010
- US-A1- 2011 108 025
- US-A1- 2012 145 148

## Description

### Introduction

This invention relates to the delivery of aerosol to patients in response to spontaneous breathing. US2011/0108025 describes an aerosol transfer device coupled to a nebuliser which generates an aerosol plume and to a patient interface. Upon inhalation by a patient ambient air is drawn into the device and flows counter-currently to the aerosol plume. However, the device is relatively large and cumbersome for use and complex to manufacture. It is also restricted to particular uses.

US 5617844 A discloses an aerosol delivery device comprising a housing defining a chamber, the housing having: a base; a top; a main body extending between the base and the top; an air inlet closed by an inlet valve, the air inlet being located adjacent to the base of the housing; a patient port for receiving a mouthpiece or a face mask; and an aerosol port, the inlet valve being breath actuatable for movement between an inspiration configuration in which the inlet valve is open and an exhalation configuration in which the inlet valve is closed, wherein the housing comprises a boss extending from the base of the housing and being spaced-apart inwardly of the main body of the housing to define a reception space.

### Statements of Invention

According to the invention there is provided an aerosol delivery device comprising a housing defining a chamber, the housing having:-
a base;
a top;
a main body extending between the base and the top;
an air inlet closed by an inlet valve, the air inlet being located adjacent to the base of the housing;
a patient port for receiving a mouthpiece or a face mask; and
an aerosol port for receiving a vibrating mesh aerosol generating device, the aerosol port being located in a side of the main body of the housing for delivery of aerosol into the chamber between the inlet valve and the patient port,
the inlet valve being breath actuatable for movement between an inspiration configuration in which the inlet valve is open and an exhalation configuration in which the inlet valve is closed.

According to the invention the housing comprises a boss extending from the base of the housing and being spaced-apart inwardly of the main body of the housing to define a reception space.

According to the invention the inlet valve is mounted to the boss for movement between the open and closed configurations. The boss may comprise a raised region against which a portion of the inlet valve is seated. The raised region may be defined by a rim which extends at least partially around the boss.

In one embodiment the housing comprises an oxygen supply port for connection to a supply of oxygen. The oxygen supply port may be normally closed by the inlet valve. In one case the oxygen supply port is located within the margins of the boss.

According to the invention a longitudinal axis through a center of the aerosol inlet port is substantially at right angles with respect to a longitudinal axis through the main body of the housing.

According to the invention a longitudinal axis through a center of the patient port is offset from a longitudinal axis through a center of the inlet. The main body of the housing comprises according to the invention a tapered transition section to the patient port.

The invention also provides an aerosol delivery system comprising an aerosol deliver device of the invention and a mouth piece or a face mask for connection to the patient port.

In a preferred embodiment the mouthpiece or face mask comprises an exhaust outlet closed by an exhaust valve, the inlet and exhaust valves being breath actuated from an inspiration configuration in which the inlet valve is open and the exhaust valve is closed to an exhalation configuration in which the inlet valve is closed and the exhaust valve is open.

In one case a longitudinal axis through a center of the patient port of the mouth piece subtends on angle of from 0° to 90° with a longitudinal axis of the main body of the housing. The angle may be approximately 60°.

In a further aspect the invention provides an aerosol delivery system further comprising a vibrating mesh aerosol generator for connection to the aerosol port for delivery of aerosol into inspiration gas flowing through the chamber when then inlet valve is open.

### Brief Description of the Drawings

The invention will be more clearly understood from the following description thereof, given by way of example in which:

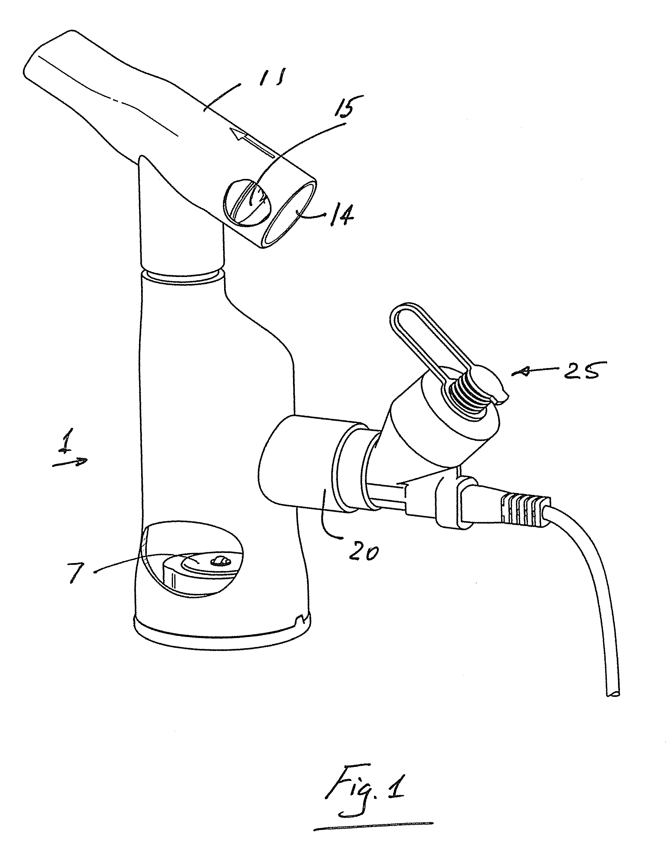

Fig. 1 is an isometric, partially cut-away view of an aerosol delivery system according to the invention;

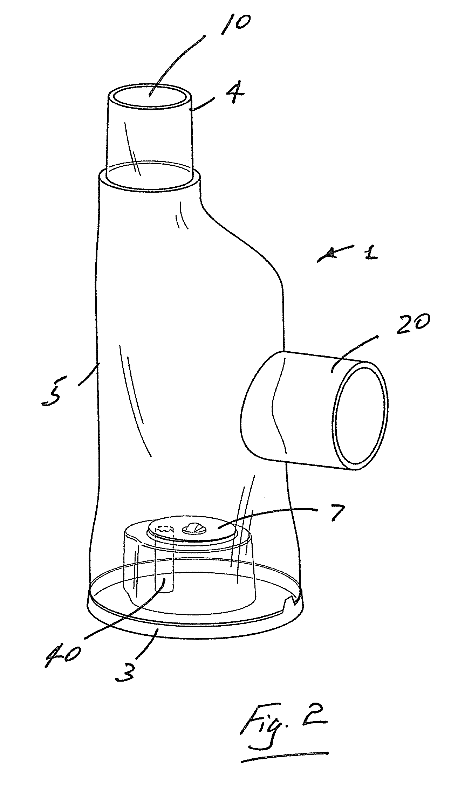

Fig. 2 is an isometric view of an aerosol delivery device of the invention forming part of the system of Fig. 1;

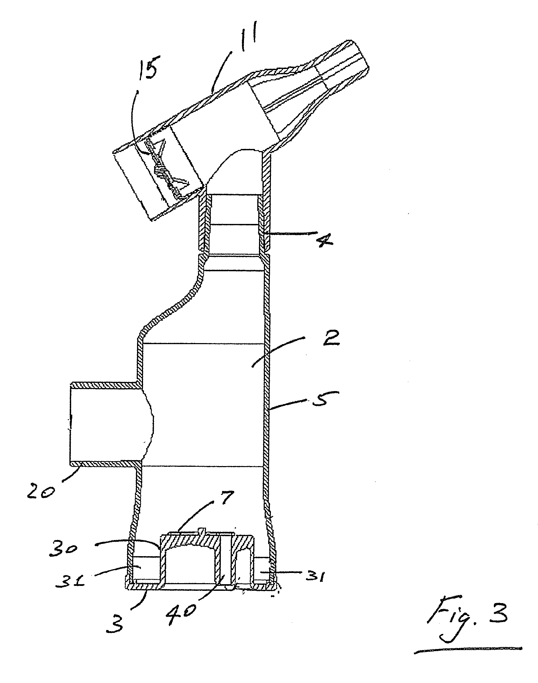

Figs. 3 and 4 are cross sectional views of the aerosol delivery system of Fig. 1;

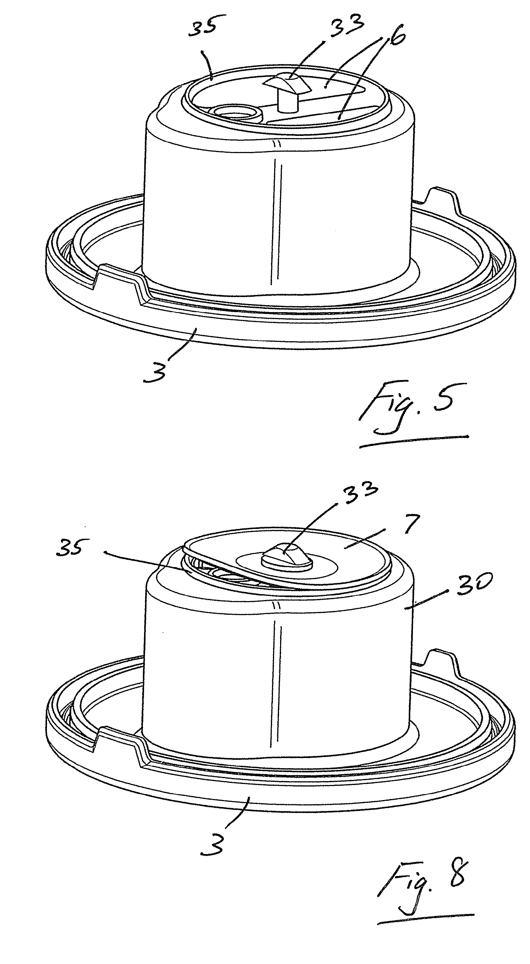

Fig. 5 is an isometric view of an air inlet end of the device with a valve removed;

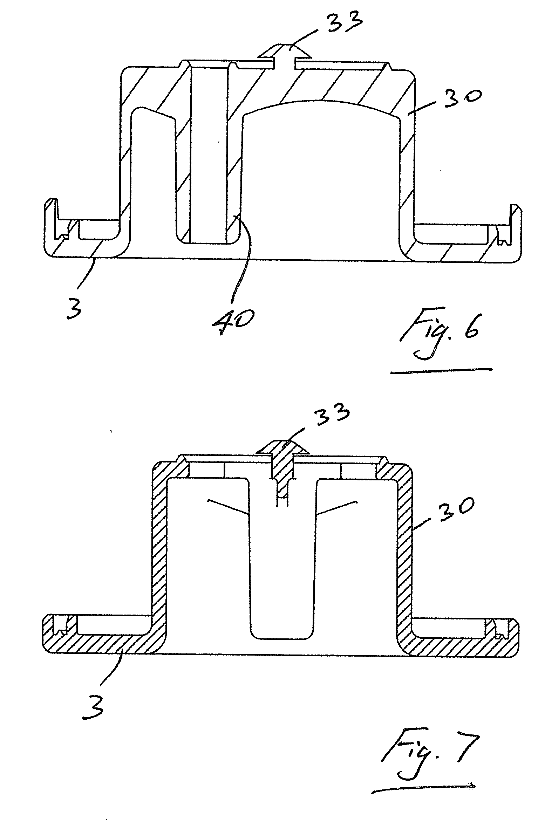

Figs. 6 and 7 are cross sectional views of the air inlet end of Fig. 5;
Fig. 8 is an isometric view of the air inlet end of the device with a valve in situ;

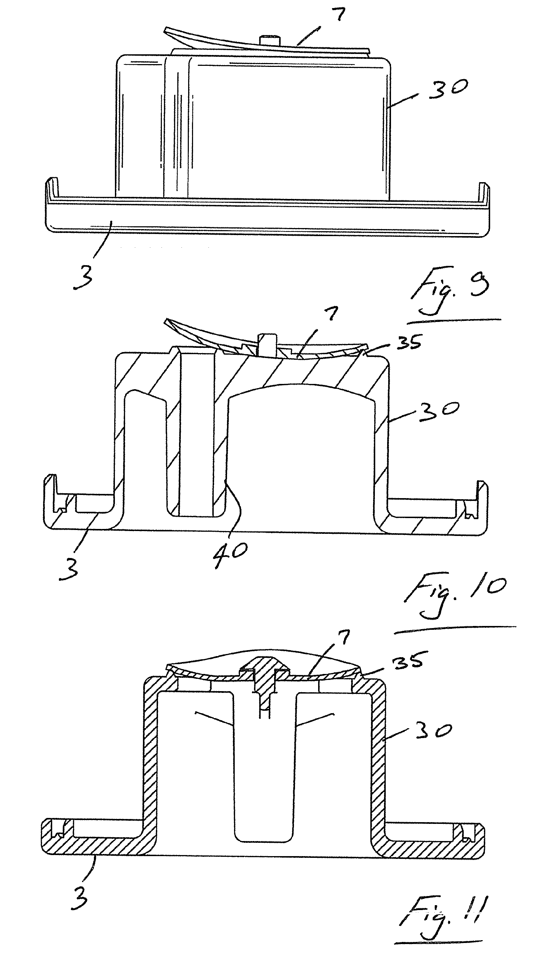

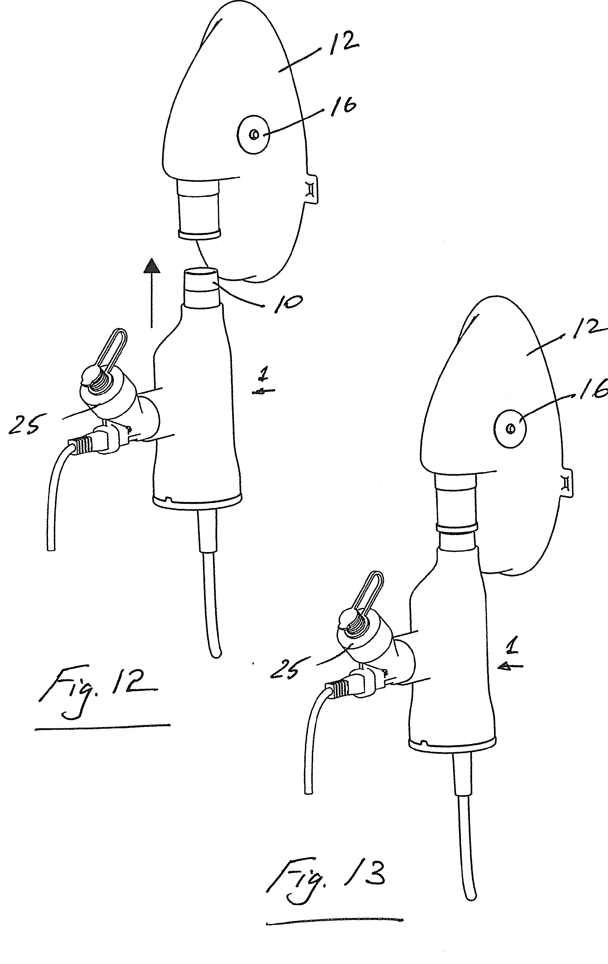

Figs. 9 to 11 are elevational and cross sectional views of the air inlet end of Fig. 8; and Figs. 12 and 13 are isometric views of the device and an associated face mask.

### Detailed Description

The invention provides a system for delivery of aerosol therapy to spontaneously breathing patients.

Referring to the drawings there is illustrated an aerosol delivery device according to the invention which comprises a housing 1 which defines a chamber 2. The housing has a base 3, a top 4 and a main body 5 extending between the base 3 and the top 4. An ambient air inlet 6 is located adjacent to the base 3 and is normally closed by an inlet valve 7.

The housing also has a patient port 10 for receiving a mouthpiece 11 or a face mask 12. The mouthpiece 11 has an exhaust outlet 14 closed by an exhaust valve 15. Similarly, the face mask 12 has an exhaust outlet closed by an exhaust valve 16. Exhaled air is exhausted through the valves 15 and 16 to prevent recirculation through the chamber 2 which would adversely affect dose efficiencies.

The housing also has an aerosol port 20 for receiving a vibrating mesh aerosol generating device 25. The aerosol port 20 is located in a side of the main body of the housing 1 for delivery of aerosol into the chamber 2 between the inlet valve 7 and the patient port 10, generally perpendicular to the flow of air through the chamber 2.

The inlet valve 7 and the exhaust valves 15, 16 are one-way breath actuated and move from an inspiration configuration in which the inlet valve 7 is open and the exhaust valve 15,16 is closed to an exhalation configuration in which the inlet valve 7 is closed and the exhaust valve 15,16 is open.

The housing 1 comprises a boss 30 extending upwardly from the base 3. The boss is spaced-apart inwardly of the main body 5 of the housing 1 to define a reception space or well 31. This facilitates collection of any rain-out within the chamber 2. The inlet valve 7 is of a flexible polymeric material such as Elastosil R401-40 (Wacker, Munich, Germany) and has a receiver for mounting to a mounting element 33. The valve 7 is movable relative to the boss 30 between the open and closed configuration. The boss 30 also has a raised region which in this case is defined by a rim 35 which extends around the boss 30 to lift one section of the valve 7. This assists in preventing adhesion between the valve 7 and the boss 30 and facilitates opening of the valve even if the inhalation force applied is low.

The housing 2 also has an oxygen supply port 40 for connection to a supply of supplemental oxygen. In this case the oxygen supply port 40 is located in the base 3 of the device within the margins of the boss 30 and is normally closed by the inlet valve 7. Thus, the inlet valve occludes the oxygen port when no oxygen flow is connected, thus maximising device efficiency. When an oxygen supply is connected the valve 7 opens. This arrangement avoids the necessity for a separates cap or valve on the oxygen supply port and diffuses oxygen flow entering the chamber 2 which improves the efficiency of the device.

It will be noted that a longitudinal axis through a center of the aerosol delivery port 20 is substantially at right angles with respect to a longitudinal axis through the main body of the housing 1. This feature assists in reducing aerosol impaction and therefore maximises dose efficiency during changes in flow direction associated with inhalation and exhalation. It also facilitates nebuliser placement for cable management and device usability.

A longitudinal axis through a center of the patient port 10 is offset from a longitudinal axis through a center of the air inlet. This feature also assists in reducing aerosol impaction and therefore maximises dose efficiency. This optimium placement also minimises device size and therefore maximises usability.

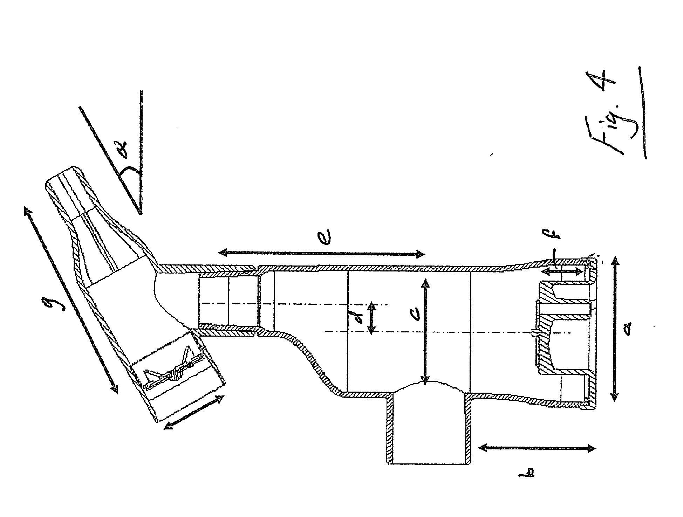

The main body of the housing 1 comprises a tapered transition section to the patient port 10. This provides minimum resistance to flow and minimises rain-out whilst maximising efficiency. Referring in particular to Fig. 4, it will also be noted that a longitudinal axis through a center of the patient port of the mouth piece subtends an angle α of from about 0° to about 90°, in this case approximately 60° to the main body of the housing. This also maximises dose efficiency. Referring in particular to Fig. 4 the dimensions a to h are important in optimising device efficiency. The approximate values for these dimensions are as follows.
a : range 40 - 60 optimum approximately 46 mm
b : range 22 - 66 optimum approximately 39 mm
c : range 25 - 45 optimum approximately 37 mm
d : range 0 - 10 optimum approximately 9 mm
e : range 50 - 90 optimum approximately 67mm
f : range 17 - 25 optimum approximately 17mm
g : range 70 - 90 optimum approximately 70 mm
h : range 20 - 30 optimum approximately 24 mm

In the invention high efficiency is achieved by:
Chamber design (i.e. diameter, length, etc.)
Neb positioning (i.e. perpendicular to chamber main axis, distance between neb and opposing wall and distance between neb and inlet valve)
Valves configuration to control the flow of air through the device
Angle of mouthpiece to chamber
Alignment of chamber outlet with bottom wall
Use of inlet valve to diffuse flow oxygen into the device

Rainout management is achieved by:
Inlet valve is located in a raised position ensure it does not contact rainout
Inlet valve seals on raised rim to reduce adhesion due to rainout
Inlet valve seal design to lift one section of valve to reduce the pressure differential required to open valve

The system can be used with or without supplementary oxygen.

The aerosol generator 25 is a vibrating mesh type nebuliser as described in our WO2012/046220A.

Aerosol generators comprising a vibratable member and a plate body operably coupled to the vibratable member are known, the plate body has a top surface, a bottom surface, and a plurality of apertures extending from the top surface to the bottom surface. The apertures may be tapered such that when a liquid is supplied to one surface and the aperture plate is vibrated using the vibratable member, liquid droplets are ejected from the opposite surface. Details of such known systems are described for example in US6,235,177, US2007/0023547A, and US7,066,398.

The invention may be used to provide treatments for a variety of ailments using a variety of aerosolisable medicaments. The ailments may include pulmonary ailments such as ventilator-associated pneumonia, hospital-acquired pneumonia, community-acquired pneumonia, asthma, cystic fibrosis, mycobacterial infection, mucociliary clearance conditions, bronchitis, staph infection, fungal infections, viral infections, tuberculosis, protozoal infections, emphysema, hereditary emphysema, Chronic Obstructive Pulmonary Disease (COPD) and acute exacerbation of COPD, among others. The aerosolizable medicaments used to treat the ailments may include antitrypsins (such as alpha-1 antitrypsin), antibiotics, anti-infectives, antivirals, anti-oxidants, epithelium sodium channel blockers, bronchodilators, beta-antagonists (short and long acting)

corticosteroids, leukotrienes, protease inhibitors, surfactants, and vaccines, among other medicaments. The ailments may further include non-pulmonary-related, such as systemic conditions, such as diabetes, cancer, immune diseases, cardiovascular conditions, metabolic diseases and the like.

The invention may be used in a method of treating a patient by administering to the patient any desirable nebulised dose of aerosol.

In some cases the method of treating a patient involves administering to the patient a discrete nebulised dose of aerosol comprising from 0.05 mL to about 50 mL of a medicament or greater than 50 mL when administering continuous aerosol therapy.

Also provided are methods of treatment by administering to a patient an aerosolised formulation comprising an anti-infective dissolved in an aqueous solution that is adjusted to a pH between about 3.0 and 10.5.

In some cases the medicament is administered continuously.

In other cases the medicament is administered intermittently.

The systems are configurable to administer aerosolised medicament, such as an anti-infective, to a spontaneous-breathing patient.

Substantially all of the device may be reused for multiple treatments with a single patient before disposing thereof.

The device may be used for only a single patient, then disposed.

A filter can be positioned at the exhaust outlet to capture exhausted drug.

The invention is not limited to the embodiments hereinbefore described, which may be varied in construction and detail. The scope of the invention is defined by the appended claims.

## Claims

1. An aerosol delivery device comprising a housing (1) defining a chamber (2), the housing (1) having:-
a base (3);
a top (4);
a main body (5) extending between the base (3) and the top (4);
an air inlet (6) closed by an inlet valve (7), the air inlet (6) being located adjacent to the base (3) of the housing (1);
a patient port (10) for receiving a mouthpiece (11) or a face mask (12); and
an aerosol port (20) for receiving a vibrating mesh aerosol generating device (25),
the inlet valve (7) being breath actuatable for movement between an inspiration configuration in which the inlet valve (7) is open and an exhalation configuration in which the inlet valve (7) is closed,
wherein:
the aerosol port (20) is located in a side of the main body (5) of the housing for delivery of aerosol into the chamber (2) between the inlet valve (7) and the patient port (10);
a longitudinal axis through a center of the aerosol port (20) is substantially at right angles with respect to a longitudinal axis through the main body (5) of the housing;
a longitudinal axis through a center of the patient port (10) is offset from a longitudinal axis through a center of the air inlet (6);
the main body (5) of the housing (1) comprises a tapered transition section to the patient port (10);
the housing (1) comprises a boss (30) extending from the base (3) of the housing and being spaced-apart inwardly of the main body (5) of the housing to define a reception space (31);
and the inlet valve (7) is mounted to the boss (30) for movement between the open and closed configurations.

2. An aerosol delivery device as claimed in claim 1 wherein the boss (30) comprises a raised region (35) against which a portion of the inlet valve (7) is seated.

3. An aerosol delivery device as claimed in claim 2 wherein the raised region (35) is defined by a rim which extends partially around the boss (30).

4. An aerosol delivery device as claimed in any of claims 1 to 3 wherein the housing comprises an oxygen supply port (40) for connection to a supply of oxygen.

5. An aerosol delivery device as claimed in claim 4 wherein the oxygen supply port (40) is normally closed by the inlet valve (7).

6. An aerosol delivery device as claimed in claim 4 or 5 wherein the oxygen supply port (40) is located within the margins of the boss (30).

7. An aerosol delivery system comprising an aerosol delivery device as claimed in any of claims 1 to 6 and a mouth piece (11) for connection to the patient port (10).

8. An aerosol delivery system as claimed in claim 7 wherein the mouthpiece (11) comprises an exhaust outlet closed by an exhaust valve, the inlet and exhaust valves being breath actuated from an inspiration configuration in which the inlet valve is open and the exhaust valve is closed to an exhalation configuration in which the inlet valve is closed and the exhaust valve is open.

9. An aerosol delivery system as claimed in claim 7 or 8 wherein a longitudinal axis through a center of the patient port of the mouth piece (11) subtends an angle of approximately 60° with a longitudinal axis of the main body of the housing.

10. An aerosol delivery system comprising an aerosol delivery device as claimed in any of claims 1 to 6 and a face mask (12) for connection to the patient port.

11. An aerosol delivery system as claimed in claim 10 wherein the face mask (12) comprises an exhaust outlet closed by an exhaust valve, the inlet and exhaust valves being breath actuated from an inspiration configuration in which the inlet valve is open and the exhaust valve is closed to an exhalation configuration in which the inlet valve is closed and the exhaust valve is open.

12. An aerosol delivery system as claimed in any of claims 7 to 11 further comprising a vibrating mesh aerosol generator for connection to the aerosol port for delivery of aerosol into inspiration gas flowing through the chamber when the inlet valve is open.

## Patentansprüche

1. Aerosolzufuhrvorrichtung, umfassend ein Gehäuse (1), das eine Kammer (2) definiert, wobei das Gehäuse (1) Folgendes aufweist:
einen Boden (3);
einen Oberteil (4);
einen Hauptkörper (5), der sich zwischen dem Boden (3) und dem Oberteil (4) erstreckt;
einen Lufteinlass (6), der von einem Einlassventil (7) verschlossen wird, wobei sich der Lufteinlass (6) dem Boden (3) des Gehäuses (1) benachbart befindet;
eine Patientenöffnung (10) zum Aufnehmen eines Mundstücks (11) oder einer Gesichtsmaske (12); und
eine Aerosolöffnung (20) zum Aufnehmen einer Aerosolerzeugungsvorrichtung (25) mit vibrierendem Netz,
wobei das Einlassventil (7) durch Atem zur Bewegung zwischen einer Einatmungskonfiguration, in der das Einlassventil (7) offen ist und einer Ausatmungskonfiguration, in der das Einlassventil (7) geschlossen ist, betätigbar ist, wobei:
sich die Aerosolöffnung (20) in einer Seite des Hauptkörpers (5) des Gehäuses befindet, um Aerosol in die Kammer (2) zwischen dem Einlassventil (7) und der Patientenöffnung (10) zuzuführen;
eine Längsachse durch eine Mitte der Aerosolöffnung (20) im Wesentlichen rechtwinklig in Bezug auf eine Längsachse des Hauptkörpers (5) des Gehäuses ist;
eine Längsachse durch eine Mitte der Patientenöffnung (10) gegenüber einer Längsachse durch eine Mitte des Lufteinlasses (6) versetzt ist;
der Hauptkörper (5) des Gehäuses (1) einen verjüngten Übergangsabschnitt zu der Patientenöffnung (10) umfasst;
das Gehäuse (1) eine Erhebung (30) umfasst, die sich von dem Boden (3) des Gehäuses erstreckt und nach innen von dem Hauptkörper (5) des Gehäuses beabstandet ist, um einen Aufnahmeraum (31) zu definieren;
und das Einlassventil (7) zur Bewegung zwischen der offenen und der geschlossenen Konfiguration an der Erhebung (30) angebracht ist.

2. Aerosolzufuhrvorrichtung nach Anspruch 1, wobei die Erhebung (30) einen erhöhten Bereich (35) umfasst, an dem ein Abschnitt des Einlassventils (7) anliegt.

3. Aerosolzufuhrvorrichtung nach Anspruch 2, wobei der erhöhte Bereich (35) von einem Rand definiert wird, der sich teilweise um die Erhebung (30) erstreckt.

4. Aerosolzufuhrvorrichtung nach einem der Ansprüche 1 bis 3, wobei das Gehäuse eine Sauerstoffeinspeiseöffnung (40) zur Verbindung mit einer Einspeisung von Sauerstoff umfasst.

5. Aerosolzufuhrvorrichtung nach Anspruch 4, wobei die Sauerstoffeinspeiseöffnung (40) normalerweise von dem Einlassventil (7) verschlossen wird.

6. Aerosolzufuhrvorrichtung nach Anspruch 4 oder 5, wobei die Sauerstoffeinspeiseöffnung (40) innerhalb der Begrenzungen der Erhebung (30) gelegen ist.

7. Aerosolzufuhrsystem, umfassend eine Aerosolzufuhrvorrichtung nach einem der Ansprüche 1 bis 6 und ein Mundstück (11) zur Verbindung mit der Patientenöffnung (10).

8. Aerosolzufuhrsystem nach Anspruch 7, wobei das Mundstück (11) einen von einem Ausströmventil verschlossenen Ausströmauslass umfasst, wobei das Einlass- und das Ausströmventil durch Atem von einer Einatmungskonfiguration, in der das Einlassventil offen ist und das Ausströmventil geschlossen ist, zu einer Ausatmungskonfiguration, in der das Einlassventil geschlossen ist und das Ausströmventil offen ist, betätigt wird.

9. Aerosolzufuhrsystem nach Anspruch 7 oder 8, wobei eine Längsachse durch eine Mitte der Patientenöffnung des Mundstücks (11) einen Winkel von ungefähr 60° mit einer Längsachse des Hauptkörpers des Gehäuses einschließt.

10. Aerosolzufuhrsystem, umfassend eine Aerosolzufuhrvorrichtung nach einem der Ansprüche 1 bis 6 und eine Gesichtsmaske (12) zur Verbindung mit der Patientenöffnung.

11. Aerosolzufuhrsystem nach Anspruch 10, wobei die Gesichtsmaske (12) einen von einem Ausströmventil verschlossenen Ausströmauslass umfasst, wobei das Einlass- und das Ausströmventil durch Atem von einer Einatmungskonfiguration, in der das Einlassventil offen ist und das Ausströmventil geschlossen ist, zu einer Ausatmungskonfiguration, in der das Einlassventil geschlossen ist und das Ausströmventil offen ist, betätigt wird.

12. Aerosolzufuhrsystem nach einem der Ansprühe 7 bis 11, weiter umfassend einen Aerosolgenerator mit vibrierendem Netz zur Verbindung mit der Aerosolöffnung zum Zuführen von Aerosol in Einatmungsgas, das durch die Kammer strömt, wenn das Einlassventil offen ist.

## Revendications

1. Dispositif d'administration d'aérosol comportant un boîtier (1) définissant une chambre (2), le boîtier (1) ayant :
un socle (3) ;
une partie supérieure (4) ;
un corps principal (5) s'étendant entre le socle (3) et la partie supérieure (4) ;
une entrée d'air (6) fermée par une soupape d'entrée (7), l'entrée d'air (6) étant située de manière adjacente par rapport au socle (3) du boîtier (1) ;
un orifice côté patient (10) servant à recevoir un embout buccal (11) ou un masque facial (12) ; et
un orifice d'aérosol (20) servant à recevoir un dispositif générateur d'aérosol à maille vibrante (25), la soupape d'entrée (7) étant en mesure d'être actionnée par la respiration à des fins de mouvement entre une configuration d'inspiration dans laquelle la soupape d'entrée (7) est ouverte et une configuration d'expiration dans laquelle la soupape d'entrée (7) est fermée,
dans lequel :
l'orifice d'aérosol (20) est situé dans un côté du corps principal (5) du boîtier à des fins d'administration d'aérosol dans la chambre (2) entre la soupape d'entrée (7) et l'orifice côté patient (10) ;
un axe longitudinal au travers d'un centre de l'orifice d'aérosol (20) est sensiblement à angle droit par rapport à un axe longitudinal au travers du corps principal (5) du boîtier ;
un axe longitudinal au travers d'un centre de l'orifice côté patient (10) est décalé par rapport à un axe longitudinal au travers d'un centre de l'entrée d'air (6) ;
le corps principal (5) du boîtier (1) comporte une section de transition conique jusqu'à l'orifice côté patient (10) ;
le boîtier (1) comporte un bossage (30) s'étendant depuis le socle (3) du boîtier et étant espacé vers l'intérieur du corps principal (5) du boîtier afin de définir un espace de réception (31) ;
et la soupape d'entrée (7) est montée sur le bossage (30) à des fins de mouvement entre les configurations ouverte et fermée.

2. Dispositif d'administration d'aérosol selon la revendication 1, dans lequel le bossage (30) comporte une région surélevée (35) contre laquelle une partie de la soupape d'entrée (7) est posée.

3. Dispositif d'administration d'aérosol selon la revendication 2, dans lequel la région surélevée (35) est définie par un rebord qui s'étend partiellement autour du bossage (30).

4. Dispositif d'administration d'aérosol selon l'une quelconque des revendications 1 à 3, dans lequel le boîtier comporte un orifice d'alimentation en oxygène (40) à des fins de connexion sur une alimentation en oxygène.

5. Dispositif d'administration d'aérosol selon la revendication 4, dans lequel l'orifice d'alimentation en oxygène (40) est normalement fermé par la soupape d'entrée (7).

6. Dispositif d'administration d'aérosol selon la revendication 4 ou la revendication 5, dans lequel l'orifice d'alimentation en oxygène (40) est situé dans les limites des marges du bossage (30).

7. Système d'administration d'aérosol comportant un dispositif d'administration d'aérosol selon l'une quelconque des revendications 1 à 6 et un embout buccal (11) à des fins de connexion sur l'orifice côté patient (10).

8. Système d'administration d'aérosol selon la revendication 7, dans lequel l'embout buccal (11) comporte une sortie d'échappement fermée par une soupape d'échappement, les soupapes d'entrée et d'échappement étant actionnées par la respiration pour passer d'une configuration d'inspiration, dans laquelle la soupape d'entrée est ouverte et la soupape d'échappement est fermée, à une configuration d'expiration, dans laquelle la soupape d'entrée est fermée et la soupape d'échappement est ouverte.

9. Système d'administration d'aérosol selon la revendication 7 ou la revendication 8, dans lequel un axe longitudinal au travers d'un centre de l'orifice côté patient de l'embout buccal (11) sous-tend un angle d'approximativement 60° par rapport à un axe longitudinal du corps principal du boîtier.

10. Système d'administration d'aérosol comportant un dispositif d'administration d'aérosol selon l'une quelconque des revendications 1 à 6 et un masque facial (12) à des fins de connexion sur l'orifice côté patient.

11. Système d'administration d'aérosol selon la revendication 10, dans lequel le masque facial (12) comporte une sortie d'échappement fermée par une soupape d'échappement, les soupapes d'entrée et d'échappement étant actionnées par la respiration pour passer d'une configuration d'inspiration, dans laquelle la soupape d'entrée est ouverte et la soupape d'échappement est fermée, à une configuration d'expiration, dans laquelle la soupape d'entrée est fermée et la soupape d'échappement est ouverte.

12. Système d'administration d'aérosol selon l'une quelconque des revendications 7 à 11, comportant par ailleurs un générateur d'aérosol à maille vibrante à des fins de connexion sur l'orifice d'aérosol à des fins d'administration d'un aérosol dans un gaz d'inspiration s'écoulant au travers de la chambre quand la soupape d'entrée est ouverte.
